(19) Europäisches Patentamt
European Patent Office
Office européen des brevets

(11) Veröffentlichungsnummer: **0 147 715**
**A2**

# (12) EUROPÄISCHE PATENTANMELDUNG

(21) Anmeldenummer: **84115125.1**

(22) Anmeldetag: **11.12.84**

(51) Int. Cl.⁴: **C 07 D 239/95**
C 07 D 403/04, C 07 D 409/12
C 07 D 405/12, A 61 K 31/495
C 07 D 243/08, C 07 D 295/00

(30) Priorität: **23.12.83 DE 3346675**

(43) Veröffentlichungstag der Anmeldung:
**10.07.85 Patentblatt 85/28**

(84) Benannte Vertragsstaaten:
**AT BE CH DE FR GB IT LI LU NL SE**

(71) Anmelder: **Beiersdorf Aktiengesellschaft**
**Unnastrasse 48**
**D-2000 Hamburg 20(DE)**

(72) Erfinder: **Hansen, Werner, Dr. Dipl.-Chem.**
**Frankring 5a**
**D-2000 Hamburg 65(DE)**

(54) **Substituierte 1-(4-Amino-6, 7-dialkoxy-chinazolinyl)-4-cyclohexenyl-Derivate des Piperazins und Homopiperazins, Verfahren zu ihrer Herstellung und ihre Verwendung sowie diese Verbindungen enthaltende Zubereitungen und Zwischenprodukte.**

(57) Neue substituierte 1-(4-Amino-6,7-dialkoxy-chinazolinyl)-4-cyclohexenyl-Derivate des Piperazins und Homopiperazins der allgemeinen Formel I

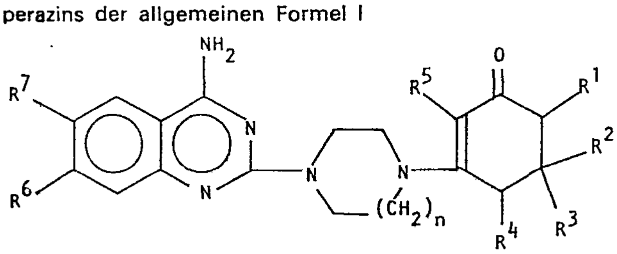

in der

$R^1$, $R^2$, $R^3$, $R^4$ und $R^5$, die gleich oder verschieden sein können, Wasserstoff, geradkettige oder verzweigte Alkylgruppen mit jeweils 1 bis 6 Kohlenstoffatomen, cyclische aliphatische Reste mit jeweils 5 bis 7 Kohlenstoffatomen, aromatische Reste oder substituierte aromatische Reste, die gleich oder verschieden mit Halogen, niederen Alkyl- oder Alkoxygruppen mit jeweils 1 bis 4 Kohlenstoffatomen im Alkylteil, einfach oder mehrfach substituiert sein können, Arylalkylreste, Furanylreste, Thienylreste oder

$R^2$ und $R^3$ zusammen die Gruppe $-(CH_2)_a-$, worin a die Zahl 4 oder 5 ist oder

$R^3$ und $R^4$ zusammen die Gruppe $-(CH_2)_b-$, worin b die Zahl 3, 4 oder 5 ist,

$R^6$ und $R^7$, die gleich oder verschieden sein können, Alkoxygruppen mit 1 bis 4 Kohlenstoffatomen im Alkylteil, der geradkettig oder verzweigt sein kann bedeuten und

n die Zahl 1 oder 2 bedeutet, und deren tautomere Formen sowie deren Säureadditionssalze und Hydrate senken den arteriellen Blutdruck und fördern den Blutfluß in Gefäßen. Sie können als Arzneimittel zur Behandlung von Hypertonie, Glaukom und Herzinsuffizienz verwendet werden.

Beiersdorf Aktiengesellschaft

Substituierte 1-(4-Amino-6,7-dialkoxy-chinazolinyl)-4-cyclohexenyl-Derivate des Piperazins und Homopiperazins, Verfahren zu ihrer Herstellung und ihre Verwendung sowie diese Verbindungen enthaltende Zubereitungen und Zwischenprodukte

Gegenstand der Erfindung sind neue substituierte 1-(4-Amino-6,7-dialkoxy-chinazolinyl)-4-cyclohexenyl-Derivate des Piperazins und Homopiperazins der allgemeinen Formel I

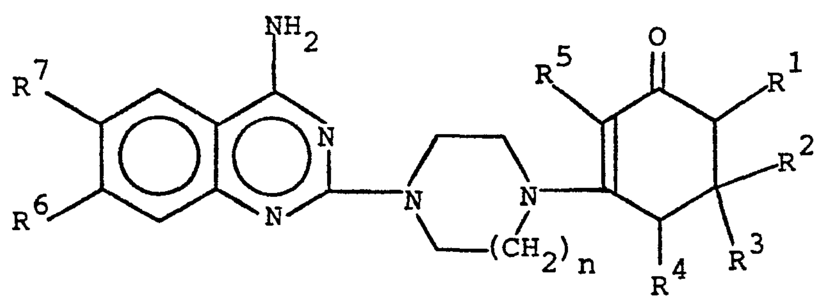

(I)

in der

$R^1$, $R^2$, $R^3$, $R^4$ und $R^5$, die gleich oder verschieden sein können, Wasserstoff, geradkettige oder verzweigte Alkylgruppen mit jeweils 1 bis 6 Kohlenstoffatomen, cyclische aliphatische Reste mit jeweils 5 bis 7 Kohlenstoffatomen, aromatische Reste oder substituierte aromatische Reste, die gleich oder verschieden mit Halogen, niederen Alkyl- oder Alkoxygruppen mit jeweils 1 bis 4 Kohlenstoffatomen im Alkylteil, einfach oder mehrfach substituiert sein können, Arylalkylreste, Furanylreste, Thienylreste oder

$R^2$ und $R^3$ zusammen die Gruppe $-(CH_2)_a-$, worin a die Zahl 4 oder 5 ist oder

$R^3$ und $R^4$ zusammen die Gruppe $-(CH_2)_b-$, worin b die Zahl 3, 4 oder 5 ist,

$R^6$ und $R^7$, die gleich oder verschieden sein können, Alkoxygruppen mit 1 bis 4 Kohlenstoffatomen im Alkylteil, der geradkettig oder verzweigt sein kann, bedeuten und

n die Zahl 1 oder 2 bedeutet, und deren tautomere Formen sowie deren Säureadditionssalze und Hydrate, Verfahren zu ihrer Herstellung und ihre Verwendung in pharmazeutischen Präparaten.

Ferner betrifft die Erfindung neue substituierte Cyclohexenderivate des Piperazins und Homopiperazins der allgemeinen Formel III

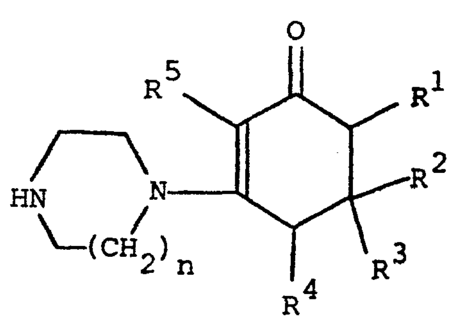

(III)

in der

$R^1$, $R^2$, $R^3$, $R^4$ und $R^5$, die gleich oder verschieden sein können, Wasserstoff, geradkettige oder verzweigte Alkylgruppen mit jeweils 1 bis 6 Kohlenstoffatomen, cyclische aliphatische Reste mit jeweils 5 bis 7 Kohlenstoffatomen, aromatische Reste oder substituierte aromatische Reste, die gleich oder verschieden mit Halogen, niederen Alkyl- oder Alkoxygruppen mit jeweils 1 bis 4 Kohlenstoffatomen im Alkylteil, einfach oder mehrfach substituiert sein können, Arylalkylreste, Furanylreste, Thienylreste oder

$R^2$ und $R^3$ zusammen die Gruppe $-(CH_2)_a-$, worin a die Zahl 4 oder 5 ist oder

$R^3$ und $R^4$ zusammen die Gruppe $-(CH_2)_b-$, worin b die Zahl 3, 4 oder 5 ist, bedeuten und

n die Zahl 1 oder 2 bedeutet,

und deren tautomere Formen sowie deren Säureadditionssalze und Hydrate, Verfahren zu deren Herstellung sowie ihre Verwendung als Zwischenprodukte für die Herstellung der erfindungsgemäßen Verbindungen der allgemeinen Formel I.

Der Einfachheit halber sind die erfindungsgemäßen Verbindungen in nur einer durch Formel I und III wiedergegebenen tautomeren Form definiert. Die Erfindung erstreckt sich jedoch auf alle tautomeren Formen der Verbindungen. Beispielsweise können Verbindungen der Formeln I und III auch in anderen tautomeren Formen auftreten, wenn der Rest $R^1$ Wasserstoff bedeutet.

Obgleich pharmazeutisch verträgliche Salze der neuen Verbindungen der Formel I und deren tautomere Formen und Hydrate bevorzugt sind, liegen alle Säureadditionssalze innerhalb des Bereichs der Erfindung. Alle Säureadditionssalze der Formeln I und III sind wertvoll zur Herstellung der Basen, selbst wenn das spezielle Salz nur als Zwischenprodukt gewünscht wird, wie z.B., wenn das Salz nur für Zwecke der Reinigung

oder Identifizierung gebildet wird, oder wenn es als ein Zwischenprodukt bei der Herstellung eines pharmazeutisch verträglichen Salzes, z.B. durch Ionenaustauschverfahrensweisen, verwendet wird.

Die erfindungsgemäßen Verbindungen können asymmetrische Kohlenstoffatome enthalten. Daher sind auch die verschiedenen optischen Isomeren sowie die Diastereoisomeren Gegenstand dieser Erfindung ebenso wie die Hydrate und Additionssalze dieser Verbindungen mit Säuren. Racemate können nach an sich bekannten Methoden in ihre optischen Antipoden aufgetrennt werden, beispielsweise durch Verwendung optisch aktiver Säuren wie Weinsäure, Kampfersulfonsäure oder Dibenzoylweinsäure, oder als Ester oder Äther mit optisch aktiven Komponenten oder über Harnstoffeinschlußverbindungen.

Bevorzugte Reste $R^1$ bis $R^5$ sind Alkylgruppen, vorzugsweise Methyl-, Ethyl-, n-Propyl- und Isopropylgruppen, Cyclohexyl-, Phenyl- und Naphthylgruppen. Substituierte aromatische Reste, insbesondere Phenylgruppen, besitzen vorzugsweise einen oder zwei gleiche oder verschiedene der genannten Substituenten, und zwar insbesondere Fluor, Chlor, Brom, Methyl, Ethyl, Methoxy und Ethoxy.

Arylalkylreste (Aralkyl) sind vorzugsweise solche mit 1 bis 4, insbesondere 1 oder 2 Kohlenstoffatomen im Alkylteil und als Aryl wird Phenyl besonders bevorzugt. Ein bevorzugter Phenylalkylrest ist die Benzylgruppe.

Bedeuten $R^2$ und $R^3$ zusammen die Gruppe $-(CH_2)_a-$ werden Spiro-cyclopentan- und Spiro-cyclohexan-Verbindungen erhalten. Bevorzugt sind die Spiro-cyclohexan-Verbindungen.

Bedeuten $R^3$ und $R^4$ zusammen die Gruppe $-(CH_2)_b-$, so ist b vorzugsweise die Zahl 4.

Die Reste $R^6$ und $R^7$ sind vorzugsweise Methoxy und Ethoxy. Ganz besonders bevorzugt werden die 6,7-Dimethoxyverbindungen.

Es werden weiterhin Verbindungen der Formeln I und III bevorzugt, in der n 1 oder 2 ist, die mit $R^2$ und $R^3$ in der 5-Stellung des Cyclohexenonylrings einfach oder zweifach substituiert sind, wobei $R^2$ und/oder $R^3$ vorzugsweise Alkyl, bevorzugt Methyl, Ethyl, n-Propyl und Isopropyl, insbesondere aber Dimethyl, oder $R^2$ und $R^3$ zusammen die Gruppe $-(CH_2)_a-$ bedeuten.

Neben den in den Beispielen genannten Verbindungen werden Verbindungen der allgemeinen Formel I mit einem therapeutischen Effekt nachstehend aufgeführt:

1-(4-Amino-6,7-dimethoxy-chinazolin-2-yl)-4-(3-oxo-5-methyl-5-äthyl-1-cyclohexen-1-yl)-piperazin

1-(4-Amino-6,7-dimethoxy-chinazolin-2-yl)-4-(3-oxo-5,5-diäthyl-1-cyclohexen-1-yl)-piperazin

1-(4-Amino-6,7-dimethoxy-chinazolin-2-yl)-4-(3-oxo-5-methyl-5-(n-propyl)-1-cyclohexen-1-yl)-piperazin

1-(4-Amino-6,7-dimethoxy-chinazolin-2-yl)-4-(3-oxo-5-methyl-5-isopropyl-1-cyclohexen-1-yl)-piperazin

1-(4-Amino-6,7-dimethoxy-chinazolin-2-yl)-4-(3-oxo-5,5-diisopropyl-1-cyclohexen-1-yl)-piperazin

1-(4-Amino-6,7-dimethoxy-chinazolin-2-yl)-4-(3-oxo-5-äthyl-1-cyclohexen-1-yl)-piperazin

1-(4-Amino-6,7-dimethoxy-chinazolin-2-yl)-4-(3-oxo-5-äthyl-6-äthyl-1-cyclohexen-1-yl)-piperazin

1-(4-Amino-6,7-dimethoxy-chinazolin-2-yl)-4-(3-oxo-5-isopropyl-6-methyl-1-cyclohexen-1-yl)-piperazin

1-(4-Amino-6,7-dimethoxy-chinazolin-2-yl)-4-(3-oxo-5-isopropyl-6-äthyl-1-cyclohexen-1-yl)-piperazin

1-(4-Amino-6,7-dimethoxy-chinazolin-2-yl)-4-(3-oxo-5-isopropyl-6-isopropyl-1-cyclohexen-1-yl)-piperazin

1-(4-Amino-6,7-dimethoxy-chinazolin-2-yl)-4-(3-oxo-6-äthyl-1-cyclohexen-1-yl)-piperazin

Die folgenden Verbindungen der allgemeinen Formel I und deren Salze und Hydrate mit hohem therapeutischen Effekt werden besonders bevorzugt, und zwar in der Form der Racemate sowie in der Form optisch aktiver Isomeren und Diastereoisomeren:

1-(4-Amino-6,7-dimethoxy-chinazolin-2-yl)-4-(3-oxo-5,5-dimethyl-1-cyclohexen-1-yl)-piperazin

1-(4-Amino-6,7-dimethoxy-chinazolin-2-yl)-4-(3-oxo-5-methyl-1-cyclohexen-1-yl)-piperazin

1-(4-Amino-6,7-dimethoxy-chinazolin-2-yl)-4-(3-oxo-5-isopropyl-1-cyclohexen-1-yl)-piperazin

1-(4-Amino-6,7-dimethoxy-chinazolin-2-yl)-4-(3-oxo-5-phenyl-6-methyl-1-cyclohexen-1-yl)-piperazin

1-(4-Amino-6,7-dimethoxy-chinazolin-2-yl)-4-(3-oxo-5,5-dimethyl-6-methyl-1-cyclohexen-1-yl)-piperazin

1-(4-Amino-6,7-dimethoxy-chinazolin-2-yl)-4-(3-oxo-5,5-dimethyl-1-cyclohexen-1-yl)-(1,4-diaza-cycloheptan)

1-(4-Amino-6,7-dimethoxy-chinazolin-2-yl)-4-(3-oxo-5-methyl-6-methyl-1-cyclohexen-1-yl)-piperazin

1-(4-Amino-6,7-dimethoxy-chinazolin-2-yl)-4-(3-oxo-5-thienyl(2)-1-cyclohexen-1-yl)-piperazin

Die erfindungsgemäßen Verbindungen der Formel I und ihre Säureadditionssalze besitzen wertvolle pharmakologische Eigenschaften. Sie eignen sich zur Senkung des arteriellen Blutdrucks und zur Förderung des Blutflusses in peripheren Gefäßen wie auch Coronargefäßen. Sie haben sich als wirksame Regulatoren des cardiovasculären Systems erwiesen und können insbesondere zur Behandlung der Hypertonie eingesetzt werden. Weiterhin sind sie zur Behandlung der Herzinsuffizienz und zur Senkung des Augeninnendruckes geeignet. Auffällig ist ihre relativ lange Wirkdauer und ihre große Selektivität zu den $alpha_1$-Rezeptoren.

Die erfindungsgemäßen Verbindungen der Formel I besitzen diese hervorragenden Eigenschaften gegenüber strukturell ähnlichen Verbindungen in nicht vorhersehbarer Weise.

Insbesondere zeichen sie sich durch eine langanhaltende antihypertensive Wirkung an Ratten, Kaninchen und Katzen aus. Hierzu werden äußerst geringe Dosen von 1 bis 100 µg/kg Körpergewicht benötigt.

Beim Menschen sind zur Senkung des arteriellen Blutdruckes 0,01 bis 50 mg/Tag, insbesondere 0,1 bis 5 mg/Tag, geeignet. Diese Dosierung ist auch zur Behandlung der Herzinsuffizienz geeignet.

Die erfindungsgemäßen Verbindungen und deren Additionssalze können weiterhin, infolge ihrer hoch selektiven alpha-sympatholytischen Eigenschaften, zur Senkung des erhöhten Augeninnendruckes (Glaukom) eingesetzt werden. Die Dosierungen betragen bei der bevorzugten Konzentration von 0,1 bis 10 mg/ml, entsprechend einer 0,01 bis 1%igen wäßrigen Lösung, vorzugsweise mehrere Tropfen pro Auge zweimal bis dreimal täglich.

Gemäß der Erfindung werden pharmazeutische Zusammensetzungen geschaffen, die eine Verbindung der Formel I oder deren pharmazeutisch verträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende Präparate können ein oder mehrere Zusätze, wie Süßungsmittel, Aromatisierungsmittel, Farbstoffe und Konservierungsmittel, enthalten. Tabletten können den Wirkstoff mit üblichen, pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Äthanol, Propylenglycol, Äther des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyäthylenstearat und Polyoxyäthylensorbitanmonooleat, und Konservierungsmitteln wie Äthylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in

einer Menge von 0,1 bis 50 mg.

Die neuen Verbindungen der allgemeinen Formel I sind nach den folgenden Verfahren erhältlich:

Ein erstes Verfahren zur Herstellung der Verbindungen der allgemeinen Formel I ist dadurch gekennzeichnet, daß man Chinazolin-Derivate der allgemeinen Formel II

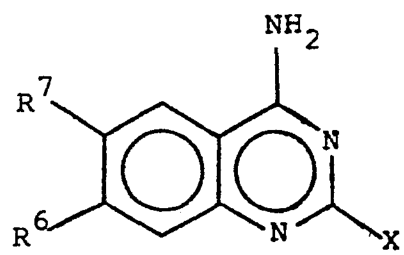

(II)

in der X Halogen, vorzugsweise Chlor oder Brom oder Alkylmercapto, vorzugsweise Methylmercapto ist und $R^6$ und $R^7$ die oben angegebene Bedeutung haben, mit Cyclohexenonderivaten des Piperazins oder Homopiperazins der allgemeinen Formel III umsetzt,

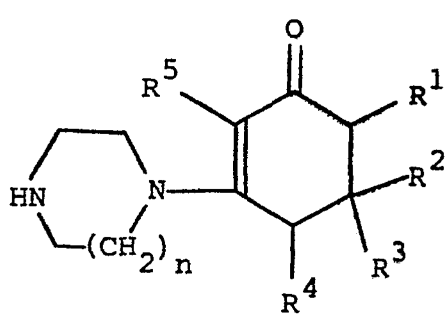

(III)

in der $R^1$ bis $R^5$ und n die obengenannte Bedeutung haben.

Die Umsetzung wird in geeigneten Lösungs- oder Dispergiermitteln wie Toluol, Xylol, halogenierten Kohlenwasserstoffen, vorzugsweise Chlorbenzol, oder Alkoholen, vorzugsweise Isoamylalkohol vorgenommen. Die Umsetzung erfolgt bei der Siedetemperatur des Lösungsmittels, vorzugsweise bei einer Temperatur von 80 - 150°C. Das Molverhältnis der Reaktionspartner II und III beträgt 1 : 1. Vorzugsweise wird ein Überschuß von 10% der Verbindung III genommen. Man kann auch mit säurebindenden Mitteln, wie Pottasche oder Triäthylamin arbeiten.

Die Herstellung der Ausgangsverbindungen der Formel II ist bekannt oder kann analog bekannten Verfahren der Literatur erfolgen (z.B. J. Chem. Soc., London, 1948, Seite 1764; US-Patentschrift 3 511 836; J. med. Chem. 20, 148 (1977)). Verbindungen mit X = S-Alkyl in der Formel II sind ebenfalls bekannt (DE-OS 1 620 138). Die Zwischenprodukte der Formel III sind neu.

Ein zweites Verfahren zur Herstellung der Verbindungen der allgemeinen Formel I geht von den Chinazolinderivaten der allgemeinen Formel II mit der oben angegebenen Bedeutung von X, $R^6$ und $R^7$ aus, die mit Piperazin oder Homopiperazin (1,4-Diazacycloheptan) der allgemeinen Formel IV in bekannter Weise (analog DE-OS 16 20 138 und US-Patent 4 001 237) umgesetzt werden. Die Verbindungen der Formel IV sind mit einer Schutzgruppe Q maskiert, beispielsweise mit einer Acylgruppe, vorzugsweise der leicht abspaltbaren Formylgruppe oder der Carbalkoxygruppe. Die Schutzgruppe läßt sich im ersten Fall sauer und im zweiten Fall zweckmäßigerweise zunächst alkalisch und dann sauer hydrolytisch von dem zunächst erhaltenen Umsetzungsprodukt der Formel V abspalten und man erhält das Umsetzungsprodukt der Formel VI.

Als Schutzgruppe gegen eine Reaktion der zweiten Iminogruppe des Piperazins oder Homopiperazins kann z.B. auch Bromwasserstoffsäure im Verhältnis 1 : 1 zum Amin herangezogen werden, so daß das Piperazin oder Homopiperazin als Monohydrobromid vorliegt:

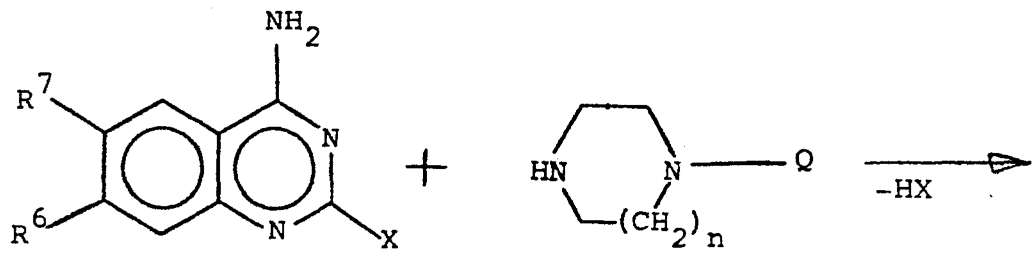


(II) (IV)

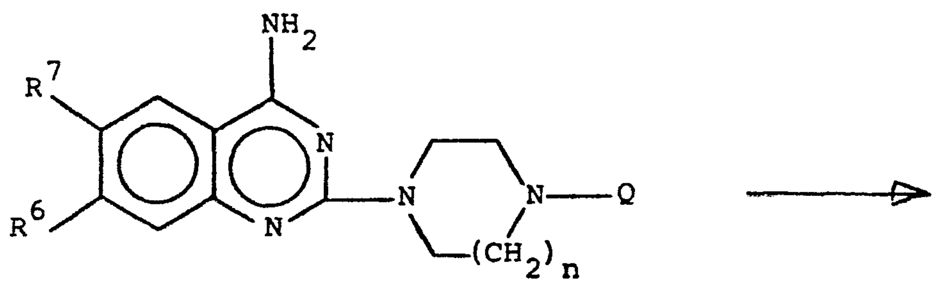

(V)

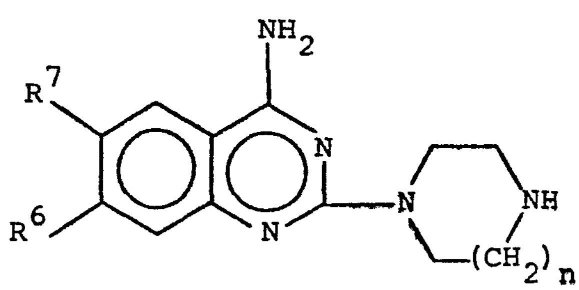

(VI)

Das zweite Verfahren zur Herstellung der Verbindungen der allgemeinen Formel I gemäß Anspruch 1 ist dadurch gekennzeichnet, daß man Verbindungen der allgemeinen Formel VI

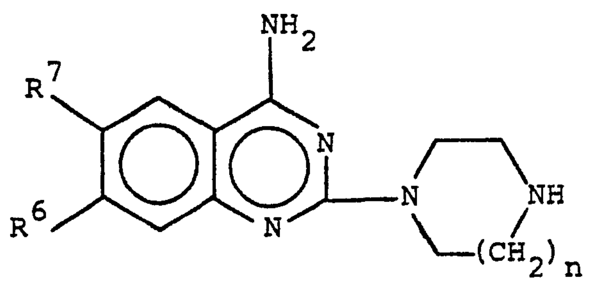

(VI)

in der $R^6$, $R^7$ und n die obengenannte Bedeutung haben, mit 1,3-Dioxocyclohexanen der allgemeinen Formel VII

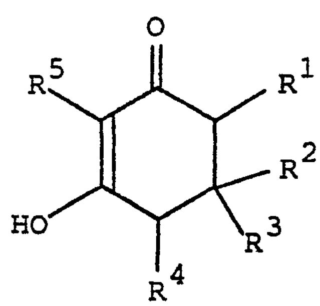

(VII)

umsetzt, in der $R^1$ bis $R^5$ die oben angegebene Bedeutung haben.

Die Kondensation erfolgt in einem Lösungsmittel bei erhöhter Temperatur, vorzugsweise in siedendem Toluol.

Die 1,3-Dioxocyclohexane der Formel VII und deren tautomere Formen sind bekannt oder können nach bekannten Verfahren erhalten werden. Ihre Herstellung erfolgt vorzugsweise nach einem der folgenden beiden Reaktionsschemen (R = Niederalkyl):

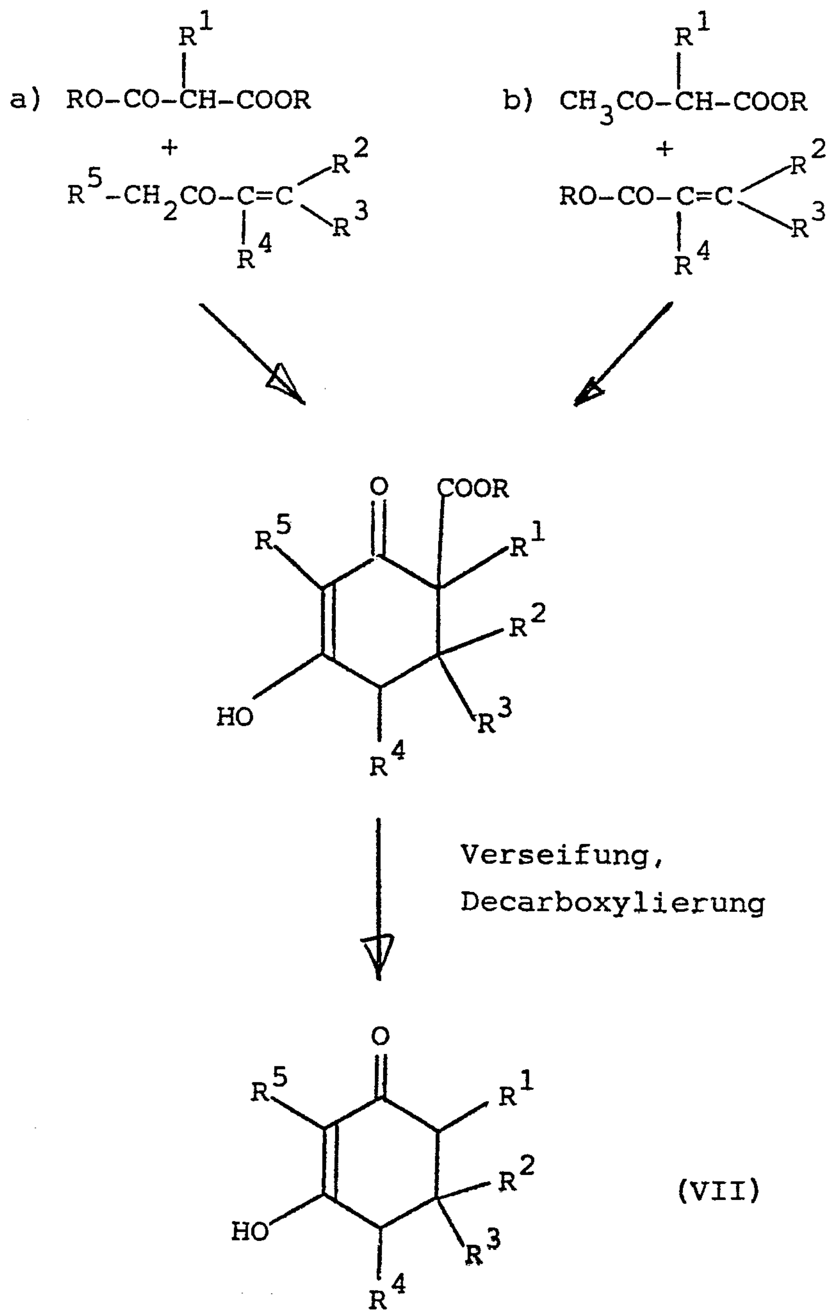


(VII)

Im Verfahren a) werden monosubstituierte oder unsubstituierte Malonester mit ungesättigten Ketonen umgesetzt, die durch Kondensation von zwei Ketonen oder einem Keton und einem Aldehyd unter alkalischen bzw. sauren Bedingungen (Aldolkondensation) erhältlich sind.

Im Verfahren b) werden monosubstituierte oder unsubstituierte ß-Ketosäureester mit substituierten Acrylsäureestern zur Reaktion gebracht.

Nach Verseifung und Decarboxylierung der zunächst gebildeten Reaktionsprodukte erhält man die Verbindungen gemäß Formel VII.

Die bei dem Verfahren verwendeten Ausgangsverbindungen sind bekannt oder können nach bekannten Methoden hergestellt werden.

In einigen Fällen, insbesondere für die Herstellung der mit $R^4$ substituierten erfindungsgemäßen Verbindungen, lassen sich die 1,3-Dioxo-cyclohexanderivate der Formel VII oder IX auch durch Hydrierung von Resorcinderivaten VIII in Gegenwart eines Katalysators, vorzugsweise Raney-Nickel, herstellen:

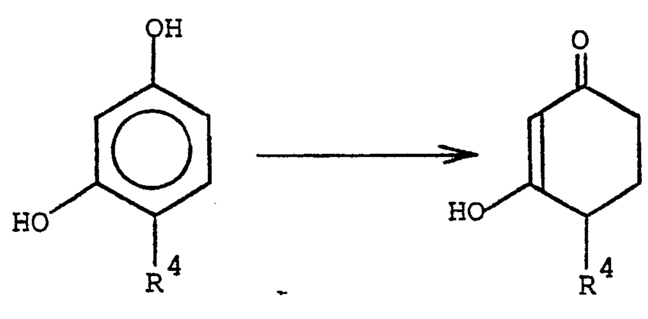

(VIII) (IX)

Die erfindungsgemäßen Zwischenprodukte der allgemeinen Formel III sind in der Literatur nicht beschrieben. Das Verfahren zur Herstellung der Verbindungen der allgemeinen Formel III mit der obengenannten Bedeutung der Reste $R^1$ bis $R^5$ und n ist dadurch gekennzeichnet, daß man Piperazin oder Homopiperazin mit Verbindungen der allgemeinen Formel VII mit der oben angegebenen Bedeutung der Reste $R^1$ bis $R^5$ kondensiert. Die Umsetzung erfolgt in einem Lösungsmittel bei erhöhter Temperatur, vorzugsweise in siedendem Toluol.

Die Verbindungen der allgemeinen Formel I können entweder als Basen oder in der Form ihrer Salze aus den Reaktionsgemischen isoliert werden. Sie lassen sich als Basen mit geeigneten anorganischen oder organischen Säuren nach bekannten Verfahren in Salze überführen.

Bevorzugt werden physiologisch verträgliche Salze. Hierfür sind als anorganische Säuren beispielsweise Halogenwasserstoffsäuren, zum Beispiel Salzsäure oder Schwefelsäure, und als organische Säuren zum Beispiel Fumarsäure, Maleinsäure, Zitronensäure und Weinsäure geeignet. Zur Herstellung wird die Lösung der Base mit der alkoholischen Lösung einer geeigneten Säure versetzt, und man erhält nach Etherzusatz das Salz.

Die folgenden Beispiele dienen zur Erläuterung der Erfindung:

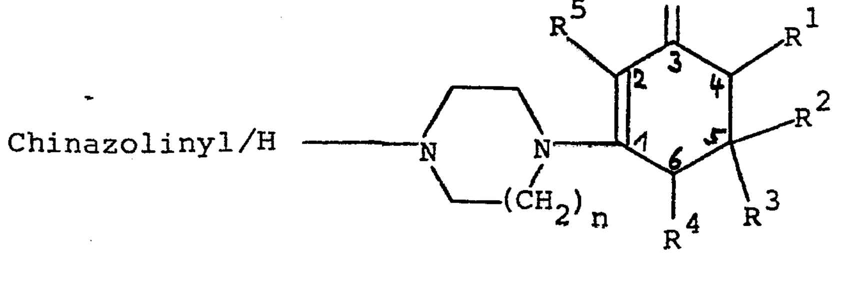

(I)/(III)

## Beispiel 1

### (3-Oxo-5,5-dimethyl-1-cyclohexen-1-yl)-piperazin

Ein Gemisch aus

28,0 g (0,2 Mol) 1,3-Dioxo-5,5-dimethyl-cyclohexan (Dimedon),

51,7 g (0,6 Mol) Piperazin, wasserfrei, und

250 ml Toluol

wird 3 Stunden am Wasserabscheider unter Rückfluß zum Sieden erhitzt. Anschließend destillieren bei einer Badtemperatur von 60°C Toluol und die Hauptmenge des überschüssigen Piperazins im Vakuum ab. Der verbleibende Rückstand wird in 300 ml Chloroform gelöst. Zur Entfernung von Piperazin und Dimedon schüttelt man die Chloroformlösung dreimal mit je 50 ml Wasser. Nach Trocknen der Chloroformlösung mit Natriumsulfat wird diese eingeengt. Der Rückstand verfestigt sich und zeigt einen Schmelzpunkt von 112 - 115°C (Ausbeute: 25,2 g = 60 %, gelbe Kristalle). Eine aus Toluol/Cyclohexan (1:2 (V)) umkristallisierte Probe (3-Oxo-5,5-dimethyl-1-cyclohexen-1-yl)-piperazin schmilzt bei 115 - 116°C.

$R_F$-Wert: 0,39.

Laufmittel: Methylalkohol/Essigester/Diäthylamin = 50 : 45 : 5 (V)

DC-Fertigplatte: Kieselgel 60 $F_{254}$ mit Konzentrierungszone, MERCK Nr. 11846.

## Beispiel 2

### (3-Oxo-5,5-dimethyl-1-cyclohexen-1-yl)-homopiperazin

Ein Gemisch aus
9,8 g (0,07 Mol) Dimedon
21,0 g (0,21 Mol) 1,4-Diaza-cycloheptan (Homopiperazin), wasserfrei, und
150 ml Toluol
wird 3 Stunden am Wasserabscheider unter Rückfluß zum Sieden erhitzt. Nach Entfernung von Toluol und überschüssigem Homopiperazin (Bad: 60°C, Vakuum) wird der verbleibende Rückstand in 20 ml Choroform gelöst und durch Chromatographie mittels einer 400 g-Kieselgel S-Säule gereinigt (Elution mit Chloroform, das steigende Anteil von Methanol (1 - 10%) enthält). Die erhaltenen gereinigten Fraktionen werden vereinigt und eingeengt. Es verbleibt ein Öl des (3-Oxo-5,5-dimethyl-1-cyclohexen-1-yl)-homopiperazins in einer Menge von 11,4 g (= 73 % Ausbeute).

Zur Darstellung des Hydrochlorids wird diese Menge in 50 ml absolutem Äthylalkohol gelöst und mit in Äthylalkohol gelöstem Chlorwasserstoff versetzt. Nach Zugabe von wasserfreiem Äther fällt das Dihydrochlorid/Monohydrat aus. Mit wasserfreiem Äther gewaschen, erhält man 10,7 g (= 49 % Ausbeute) des Hydrochlorids des (3-Oxo-5,5-dimethyl-1-cyclohexen-1-yl)-homopiperazins vom Schmelzpunkt 208 - 212°C.
$R_F$-Wert: 0,39 (Bedingungen entsprechend Beispiel 1)

## Beispiel 3

/3-Oxo-5-(o-chlorphenyl)-1-cyclohexen-1-yl7-piperazin

Ein Gemisch aus

24,0 g (0,108 Mol) 1,3-Dioxo-5-(o-chlorphenyl)-cyclohexan,

28,4 g (0,33 Mol) Piperazin, wasserfrei, und

270 ml Toluol

wird 5 Stunden am Wasserabscheider unter Rückfluß zum Sieden erhitzt. Das Toluol wird noch warm im Vakuum abdestilliert und der Rückstand in Chloroform gelöst. Diese Lösung wird zur Entfernung des überschüssigen Piperazin dreimal mit Wasser ausgeschüttelt und mit Natriumsulfat getrocknet. Die aufkonzentrierte Lösung wird mittels Säulenchromatographie gereinigt (entsprechend Beispiel 2). Ein Teil der Fraktionen enthält die Base /3-Oxo-5-(o-chlorphenyl)-1-cyclohexen-1-yl7-piperazin in reiner Form, 6,5 g vom Schmelzpunkt 159 - 160°C, ein anderer Teil noch verunreinigt. Letzterer Anteil wird über das Oxalat gereinigt: 6,2 g, weißes Salz vom Schmelzpunkt 225 - 227°C (Z). Nach Rückführung in die Base liegen weitere 5,0 g vor.
Gesamtausbeute: 11,5 g (= 36,7 %)
$R_F$-Wert: 0,38 (Bedingungen entsprechend Beispiel 1)

Beispiel 4

(3-Oxo-6-cyclohexyl-1-
cyclohexen-1-yl)-piperazin

Ein Gemisch aus
9,8 g (0,05 Mol) 1,3-Dioxo-6-cyclohexyl-cyclohexan,
12,9 g (0,15 Mol) Piperazin, wasserfrei, und
150 ml Toluol
wird wie im Beispiel 1 beschrieben zur Reaktion gebracht und aufgearbeitet. Zur Reinigung wird eine Säulenchromatographie angeschlossen (entsprechend Beispiel 2). Man erhält (3-Oxo-6-cyclohexyl-1-cyclohexen-1-yl)-piperazin als Öl in einer Menge von 9,9 g (= 76 % Ausbeute) und einem $R_F$-Wert von 0,36 (Bedingungen entsprechend Beispiel 1). Das Dihydrochlorid schmilzt bei 258 - 261°C (Z.).

Das oben eingesetzte 1,3-Dioxo-6-cyclohexyl-cyclohexan wird nach einer Vorschrift (Friedländers Fortschritte der Teerfarbenfabrikation 22, 618, Verlag Julius Springer, Berlin und DE-PS 621 915) aus einem bekannten Resorcinderivat wie folgt hergestellt:

20,0 g (0,104 Mol) 4-Cyclohexylresorcin werden in 40 ml zehnprozentiger Natronlauge gelöst und in Gegenwart von 5 g Raney-Nickel im Autoklaven bei 90° und 20 bar 5 Stunden hydriert. Das Reaktionsgemisch wird mit Alkohol aus dem Autoklaven gespült. Nach Entfernung des Katalysators wird die Lösung mit 100 ml 90-prozentiger Essigsäure langsam versetzt. Nach Zugabe von Wasser kristallisiert das Reaktionsprodukt aus. Es wird im Vakuum über NaOH getrocknet. Man erhält 12,9 g 1,3-Dioxo-6-cyclohexyl-cyclohexan vom Schmelzpunkt 137 - 140°C. Nach Umkristallisation aus Essigester schmilzt das Produkt bei 142 - 144°C (Lit.). Ausbeute: 11,1 g (= 55%).

Analog den Beispielen 1 bis 4 wurden folgende Substanzen der allgemeinen Formel III hergestellt:

| Beisp. | n | $R^1$ | $R^2$ | $R^3$ | $R^4$ | $R^5$ | Salz/Base | Schmelzp.($^oC$) | $R_F$-Wert [1)] |
|---|---|---|---|---|---|---|---|---|---|
| 5 | 1 | H | $CH_3$ | H | H | H | 2 HCl | ab 250 (Z) | 0,38 |
| 6 | 1 | H | H | H | H | $CH_3$ | 1/2 Oxals.1/2 $H_2O$ | 232-233 (Z) | 0,44 |
| 7 | 1 | H | H | $CH_3$ | $CH_3$ | H | 2 HCl | 260-265 | 0,39 |
| 8 | 1 | H | n-$C_3H_7$ | H | H | H | Base | 94-97 | 0,34 |
| 9 | 1 | H | i-$C_3H_7$ | H | H | H | Oxals.1/2 $H_2O$ | 247-249 (Z) | 0,34 |
| 10 | 1 | H | p-$CH_3O$-$C_6H_4$- | H | H | H | 1/2 Oxals. $H_2O$ | 220-221 (Z) | 0,33 |
| 11 | 1 | H | $C_6H_5$- | H | H | $CH_3$ | Base | 127-129 | 0,53 |
| 12 | 1 | H | $C_6H_5$- | H | $CH_3$ | H | Base | 139-141 | 0,37 |
| 13 | 1 | H | ——$(CH_2)_5$—— | | H | H | Base | 157-159 | 0,43 |
| 14 | 1 | H | H | H | $C_6H_5CH_2$- | H | 1/2 Oxals. | 231-233 (Z) | 0,34 |
| 15 | 1 | H | H | H | H | H | 2 HCl $H_2O$ | 239-240 (Z) | 0,31 |

1) Bedingungen entsprechend Beispiel 1

Fortsetzung:

| Beisp. | n | $R^1$ | $R^2$ | $R^3$ | $R^4$ | $R^5$ | Salz/Base | Schmelzp. ($^oC$) | $R_F$-Wert [1)] |
|---|---|---|---|---|---|---|---|---|---|
| 16 | 1 | H | H | H | n-$C_6H_{13}$ | H | 1/2 Oxals.$H_2O$ | 198-202 | 0,43 |
| 17 | 1 | H | $C_6H_5$ | H | H | H | Base | 133-134 | 0,36 |
| 18 | 1 | $CH_3$ | $CH_3$ | H | $CH_3$ | H | 2 HCl | 273-275 | 0,34 |
| 19 | 1 | H | α-Naphthyl | H | H | H | Base | 173-178 | 0,28 |
| 20 | 1 | H | $C_6H_5$ | H | $C_6H_5$ | H | Base | 230-232 | 0,34 |
| 21 | 1 | H | $CH_3$ | $CH_3$ | $CH_3$ | H | 2 HCl 1/2 $H_2O$ | 256-257 | |
| 21a | 1 | H | 2-Furanyl | H | H | H | Base | 118-119 | 0,31 |
| 21b | 1 | H | 2-Thienyl | H | H | H | 2 HCl | 273-274 | 0,55 |
| 21c | 1 | H | H | $-(CH_2)_4-$ ($R^3$ und $R^4$ zusammen) | | H | Base | 107-109 | 0,37 |

1) Bedingungen entsprechend Beispiel 1

Die Herstellung der erfindungsgemäßen Verbindungen der Formel I wird an folgenden Beispielen erläutert:

Beispiel 22

1-(4-Amino-6,7-dimethoxy-chinazolin-2-yl)-4-(3-oxo-5,5-dimethyl-1-cyclohexen-1-yl)-piperazin-dihydrochlorid-dihydrat

Ein Gemisch aus

4,8 g (0,02 Mol) 2-Chlor-4-amino-6,7-dimethoxy-chinazolin,

4,6 g (0,022 Mol) (3-Oxo-5,5-dimethyl-1-cyclohexen-1-yl)-piperazin und

80 ml Isoamylalkohol

wird 3 Stunden unter Rühren am Rückfluß gekocht. Während der Reaktionszeit fällt das Monohydrochlorid aus. Man läßt über Nacht stehen und saugt die Kristalle ab. Nach Waschen mit 40 ml Isoamylalkohol, zweimal mit je 40 ml absolutem Aceton und 40 ml absolutem Äther erhält man nach Trocknung 7,1 g des Hydrochlorids von 1-(4-Amino-6,7-dimethoxy-chinazolin-2-yl )-4-(3-oxo-5,5-dimethyl-1-cyclohexen-1-yl)-piperazin.
(78 % Ausbeute) vom Schmelzpunkt 267 - 268°C (Z).

Zur Umwandlung in die Base wird das Hydrochlorid in 140 ml Wasser gelöst und mit 35 ml konzentrierter Ammoniaklösung unter Eiskühlung versetzt. Die ausfallende Base wird zweimal mit je 120 ml Chloroform extrahiert und mit Natriumsulfat getrocknet. Nach Einengen der Chloroformlösung wird der Rückstand aus etwa 400 ml Acetonitril umkristallisiert. Man erhält die Base 1-(4-Amino-6,7-dimethoxy-chinazolin-2-yl)-4-(3-oxo-5,5-dimethyl-1-cyclohexen-1-yl)-piperazin als schwach gelb gefärbte Substanz mit einem Schmelzpunkt von 186 - 188°C.

Umwandlung der Base in das Dihydrochlorid/ Dihydrat: 10 g Base werden in 30 ml Methanol gelöst und unter Eiskühlung mit 0,5 ml konzentrierter Salzsäure versetzt. Nach vorsichtiger Zugabe von 20 ml Äther kristallisiert das Produkt aus. Man erhält 1,0 g vom Schmelzpunkt 245 - 255°C (Z).

$R_F$-Wert: 0,58; Laufmittel: $CH_3OH$ / Essigester / Diäthylamin (20:70:5 (V)) DC-Fertigplatte: Kieselgel 60 $F_{254}$, mit Konzentrationszone, Merck Nr. 11846

Beispiel 22 a

1-(4-Amino-6,7-dimethoxy-chinazolin-2-yl)-4-(3-oxo-5,5-dimethyl-1-cyclohexen-1-yl)-piperazin

Ein Gemisch aus

2,9 g (0,01 Mol) 2-Piperazino-4-amino-6,7-dimethoxy-chinazolin,

1,4 g (0,01 Mol) Dimedon und

20 ml Toluol

wird 4 Stunden am Wasserabscheider unter Rückfluß zum Sieden erhitzt. Nach dem Abkühlen der Lösung fällt das Reaktionsprodukt aus. Es wird mit Petroläther gewaschen und aus 220 ml Acetonitril umkristallisiert. Man erhält 2,6 g vom Schmelzpunkt 185 - 187°C.

$R_F$-Wert: 0,55 (Bedingungen entsprechend Beispiel 22)

Beispiel 23

1-(4-Amino-6,7-dimethoxy-chinazolin-2-yl)-4-(3-oxo-5,5-dimethyl-1-cyclohexen-1-yl)-homopiperazin-dihydrochlorid

Ein Gemisch aus

1,7 g (0,007 Mol) 2-Chlor-4-amino-6,7-dimethoxy-chinazolin,

2,2 g (0,007 Mol) (3-Oxo-5,5-dimethyl-1-cyclohexen-1-yl)-homopiperazin-dihydrochlorid-monohydrat

3,55 g (0,035 Mol) Triäthylamin und

30 ml Isoamylalkohol, wasserfrei,

wird 10 Stunden unter Rückfluß zum Sieden erhitzt. Nach Abkühlen wird das Triäthylamin-hydrochlorid abgesaugt und mit Isoamylalkohol gewaschen. Die Alkoholphasen werden bei 50°C im Vakuum eingeengt und der verbleibende Rückstand wird in 200 ml Chloroform gelöst. Nach zweimaligem Waschen mit Wasser wird die Chloroformlösung mit Natriumsulfat getrocknet und zur Trockne eingedampft. Der Rückstand läßt sich mit 20 ml absolutem Äthanol aufnehmen. Zu dieser Lösung fügt man in Äthylalkohol gelösten Chlorwasserstoff und anschließend absoluten Äther bis zur beginnenden Trübung unter Eiskühlung. Das ausfallende 1-(4-Amino-6,7-dimethoxy-chinazolin-2-yl)-4-(3-oxo-5,5-dimethyl-1-cyclohexen-1-yl)-homopiperazin-dihydrochlorid wird abgesaugt und mit absolutem Äther gewaschen.

Ausbeute: 2,1 g (60 %) vom Schmelzpunkt 259 - 261°C (Z).

Eine aus Äthanol/Methanol (2 : 1) umkristallisierte Probe schmilzt bei 260 - 261°C (Z).

$R_F$-Wert: 0,47 (Bedingungen entsprechend Beispiel 22)

## Beispiel 24

### 1-(4-Amino-6,7-dimethoxy-chinazolin-2-yl)-4-[3-oxo-5-(o-chlorphenyl)-1-cyclohexen-1-yl]-piperazin-dihydrochlorid-dihydrat

Ein Gemisch aus

2,4 g (0,01 Mol) 2-Chlor-4-amino-6,7-dimethoxy-chinazolin,

3,2 g (0,011 Mol) [3-Oxo-5-(o-chlorphenyl)-1-cyclohexen-1-yl]-piperazin und

40 ml Isoamylalkohol

wird 3 Stunden unter Rückfluß zum Sieden erhitzt (Bad: 150°C). Nach Abkühlen werden die Kristalle abgesaugt und nacheinander mit 20 ml Isoamylalkohol, zweimal mit je 20 ml absolutem Aceton und wasserfreiem Äther gewaschen. Das im Vakuum bei 40°C getrocknete 1-(4-Amino-6,7-dimethoxy-chinazolin-2-yl)-4-[3-oxo-5-(o-chlorphenyl)-1-cyclohexen-1-yl]-piperazin-hydrochlorid schmilzt bei 225 - 227°C unter Zersetzung.

5,0 g (94 % Ausbeute), gelbe Kristalle.

Zur Überführung in die Base wird das Monohydrochlorid in 20 ml Wasser aufgeschlemmt und mit 15 ml konzentrierter Ammoniaklösung unter Kühlen versetzt. Man zieht die Base dreimal mit je 200 ml Essigester aus, wäscht zweimal mit je 20 ml Wasser und trocknet die organische Phase mit Natriumsulfat. Nach dem Einengen kann die Base aus Isopropanol umkristallisiert werden. Das so gereinigte 1-(4-Amino-6,7-dimethoxy-chinazolin-2-yl)-4-[3-oxo-5-(o-chlorphenyl)-1-cyclohexen-1-yl]-piperazin wird zur Überführung in das Dihydrochlorid-dihydrat in wenig Methanol gelöst und mit 0,5 ml konzentrierter Salzsäure unter Kühlen versetzt. Die erhaltenen Kristalle schmelzen ab 200°C (Z).

$R_F$-Wert: 0,54 (Bedingungen entsprechend Beispiel 22)

## Beispiel 25

### 1-(4-Amino-6,7-dimethoxy-chinazolin-2-yl)-4-(3-oxo-5-isopropyl-1-cyclohexen-1-yl)-piperazin-dihydrochlorid-dihydrat

Ein Gemisch aus

2,4 g (0,01 Mol) 2-Chlor-4-amino-6,7-dimethoxy-chinazolin,

2,45 g (0,011 Mol) (3-Oxo-5-isopropyl-1-cyclohexen-1-yl)-piperazin und

40 ml Isoamylalkohol

wird wie in Beispiel 24 beschrieben, umgesetzt und aufgearbeitet. Ein Monohydrochlorid des 1-(4-Amino-6,7-dimethoxy-chinazolin-2-yl)-4-(3-oxo-5-isopropyl-1-cyclohexen-1-yl)-piperazins fällt in einer Menge von 3,1 g (67 % Ausbeute) und einem Schmelzpunkt von 266 - 268°C (Z) an (gelbliche Kristalle). Ebenso wie in Beispiel 24 beschrieben, wird die Base durch Alkalisieren mit Ammoniak hergestellt. Die Rohbase wird zweimal mit je 100 ml Chloroform ausgezogen und zweimal mit je 15 ml Wasser gewaschen. Nach Trocknen der Chloroformphase mit Natriumsulfat wird sie eingeengt und der verbleibende Rückstand aus 20 ml Isopropanol umkristallisiert. Die erhaltene reine Base schmilzt bei 269-271°C (Z) (weiße Kristalle). Die Überführung in das Dihydrochlorid-dihydrat erfolgt ebenso wie in Beispiel 24 beschrieben. Schmelzpunkt 240 - 243°C (u.Z.)

$R_F$-Wert: 0,54 (Bedingungen entsprechend Beispiel 22)

## Beispiel 26

4-[4-(4-Amino-6,7-dimethoxy-chinazolin-2-yl)-1-piperazinyl]-spiro[5,5]undec-3-en-2-on;

Spiro-[1-{4-(4-amino-6,7-dimethoxy-chinazolin-2-yl)-piperazin-1-yl}-3-oxo-1-cyclohexen]-5,1'-cyclohexan-dihydrochlorid-dihydrat

($R^2$ und $R^3$ zusammen = $-(CH_2)_5-$; $R^1$, $R^4$, $R^5$ = H)

Ein Gemisch aus

4,8 g (0,2 Mol) 2-Chlor-4-amino-6,7-dimethoxy-chinazolin,

5,45 g (0,022 Mol) [Spiro-(3-oxo-1-cyclohexen-1-yl)-5,1'-cyclohexan]-piperazin

(4-(1-Piperazinyl)-spiro[5,5]undec-3-en-2-on)

und 120 ml Isoamylalkohol

läßt man 3 Stunden unter Rückfluß sieden. Bereits nach

5 Minuten fällt hellgelbes Reaktionsprodukt aus. Man läßt über Nacht stehen und saugt die Kristalle ab. Sie werden nacheinander gewaschen mit 40 ml absolutem Isoamylalkohol, 40 ml absolutem Aceton und 40 ml absolutem Äther. Das Monohydrochlorid fällt in einer Menge von 8,1 g (83 %) an und schmilzt bei 277 - 278°C. Zur Darstellung der Base löst man diese Menge in 150 ml Wasser und fügt unter Eiskühlung 40 ml konzentrierte Ammoniaklösung hinzu. Die ausfallende Base wird in 300 ml Chloroform aufgenommen und mit Natriumsulfat getrocknet. Nach Abdestillieren des Lösungsmittels verbleibt ein gelber Rückstand, der aus Aceton umkristallisiert, Spiro[1-{4-(4-amino-6,7-dimethoxy-chinazolin-2-yl)-piperazin-1-yl}-3-oxo-1-cyclohexen]-5,1'-cyclohexan·$CH_3COCH_3$ in einer Ausbeute von 7,8 g ergibt. Schmelzpunkt 163 - 172°C (Z). 2,0 g dieses Produkts werden in 130 ml Methanol gelöst und mit 1 ml konzentrierter Salzsäure und anschließend mit 250 ml Äther unter Kühlen versetzt. Das Dihydrochlorid-dihydrat fällt in einer Menge von 2,0 g an.
Schmelzpunkt: 243 - 245°C (Z.).
$R_F$-Wert: 0,71 (Bedingungen entsprechend Beispiel 22)

Analog den Beispielen 22 bis 26 wurden folgende Substanzen der allgemeinen Formel I, in der $R^6$ und $R^7$ jeweils Methoxygruppen bedeuten, hergestellt:

| Beisp. | n | $R^1$ | $R^2$ | $R^3$ | $R^4$ | $R^5$ | Salz/Base | Schmelzp. ($^oC$) | $R_F$-Wert[1)] |
|---|---|---|---|---|---|---|---|---|---|
| 27 | 1 | H | $CH_3$ | H | H | H | 2 HCl $2H_2O$ | ab 200 (Z) | 0,5 |
| 28 | 1 | H | H | H | H | $CH_3$ | 2 HCl $2H_2O$ | 260-264(Z) | 0,55 |
| 29 | 1 | H | H | $CH_3$ | $CH_3$ | H | 2 HCl $2H_2O$ | ab 200 (Z) | 0,55 |
| 30 | 1 | H | $n-C_3H_7$ | H | H | H | Base | ab 180 (Z) | 0,53 |
| 31 | 1 | H | H | H | $n-C_6H_{13}$ | H | HCl | ab 270 (Z) | 0,6 |
| 32 | 1 | H | $p-CH_3O-C_6H_4-$ | H | H | H | Base | 222-224 | 0,58 |
| 33 | 1 | H | H | H | $C_6H_5-CH_2-$ | H | Base | 148-151 | 0,57 |
| 34 | 1 | H | H | H | H | H | 2 HCl $2H_2O$ | ab 180 (z) | 0,47 |
| 35 | 1 | H | H | H | Cyclohexyl | H | Base | 272-275 | 0,59 |
| 36 | 1 | H | $C_6H_5$ | H | H | H | Base $H_2O$ | 185-197 | 0,58 |

1) Bedingungen entsprechend Beispiel 22

Fortsetzung

| Beisp. | n | $R^1$ | $R^2$ | $R^3$ | $R^4$ | $R^5$ | Salz/Base | Schmelzp. (°C) | $R_F$-Wert [1] |
|---|---|---|---|---|---|---|---|---|---|
| 37 | 1 | $CH_3$ | $CH_3$ | H | $CH_3$ | H | 2 Maleins.$H_2O$ | 185-188 | 0,59 |
| 38 | 1 | H | α-Naphthyl | H | H | H | Base $H_2O$ | 175-185 | 0,75 |
| 39 | 1 | H | $C_6H_5$ | H | H | $CH_3$ | Base | 275-277 (Z) | 0,63 |
| 40 | 1 | H | $C_6H_5$ | H | $CH_3$ | H | Base $H_2O$ | 268-269 | 0,54 |
| 41 | 1 | H | $C_6H_5$ | H | $C_6H_5$ | H | Base $H_2O$ | >320 (Z) | 0,55 |
| 42 | 1 | H | $CH_3$ | $CH_3$ | $CH_3$ | H | Base 1/2 $H_2O$ | 253-255 (Z) | 0,6 |
| 43 | 1 | H | 2-Furanyl | H | H | H | Base | ab 150 (Z) | 0,88 |
| 44 | 1 | H | 2-Thienyl | H | H | H | Base $H_2O$ | 260-262 | 0,82 |
| 45 | 1 | H | H | $-(CH_2)_4-$ ($R^3$ und $R^4$ zusammen) | | H | Base 1/2 $H_2O$ | 285-286 | 0,6 |

1) Bedingungen siehe Beispiel 22

## Beispiel 44

### Herstellung von Tabletten

Tabletten, welche die nachstehend angegebenen Bestandteile enthalten, werden nach bekannten Arbeitsweisen hergestellt. Diese sind für die Behandlung von Hypertonie und Herzinsuffizienz in einer Dosierungsmenge von 1 mg zweimal täglich geeignet.

| | |
|---|---|
| 1-(4-Amino-6,7-dimethoxy-chinazolin-2-yl)-4-(3-oxo-5,5-dimethyl-1-cyclohexen-1-yl)-piperazin-dihydrochlorid-dihydrat | 1 mg |
| Lactose | 200 mg |
| Maisstärke | 25 mg |
| Magnesiumstearat | 1 mg |

Patentansprüche

1. Substituierte 1-(4-Amino-6,7-dialkoxy-chinozolinyl)-4-cyclohexenyl-Derivate des Piperazins und Homopiperazins der allgemeinen Formel I

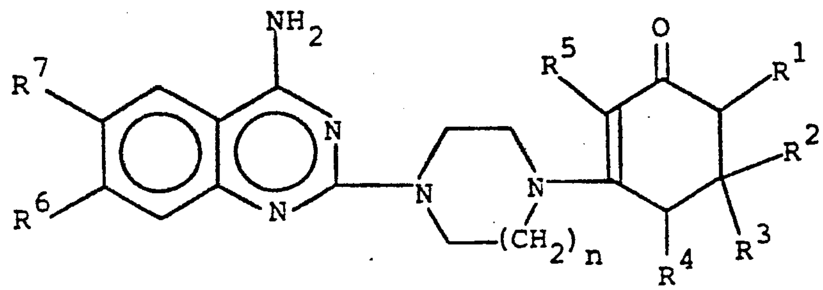

(I)

in der

$R^1$, $R^2$, $R^3$, $R^4$ und $R^5$, die gleich oder verschieden sein können, Wasserstoff, geradkettige oder verzweigte Alkylgruppen mit jeweils 1 bis 6 Kohlenstoffatomen, cyclische aliphatische Reste mit jeweils 5 bis 7 Kohlenstoffatomen, aromatische Reste oder substituierte aromatische Reste, die gleich oder verschieden mit Halogen, niederen Alkyl- oder Alkoxygruppen mit jeweils 1 bis 4 Kohlenstoffatomen im Alkylteil, einfach oder mehrfach substituiert sein können, Arylalkylreste, Furanylreste, Thienylreste oder

$R^2$ und $R^3$ zusammen die Gruppe $-(CH_2)_a-$, worin a die Zahl 4 oder 5 ist oder

$R^3$ und $R^4$ zusammen die Gruppe $-(CH_2)_b-$, worin b die Zahl 3, 4 oder 5 ist,

$R^6$ und $R^7$, die gleich oder verschieden sein können, Alkoxygruppen mit 1 bis 4 Kohlenstoffatomen im Alkylteil, der geradkettig oder verzweigt sein kann, bedeuten und

n die Zahl 1 oder 2 bedeutet,

und deren tautomere Formen

sowie deren Säureadditionssalze und Hydrate.

2. 1-(4-Amino-6,7-dimethoxy-chinazolin-2-yl)-4-(3-oxo-5,5-dimethyl-1-cyclohexen-1-yl)-piperazin

3. 1-(4-Amino-6,7-dimethoxy-chinazolin-2-yl)-4-(3-oxo-5-isopropyl-1-cyclohexen-1-yl)-piperazin

4. 1-(4-Amino-6,7-dimethoxy-chinazolin-2-yl)-4-(3-oxo-5-thienyl(2)-1-cyclohexen-1-yl)-piperazin

5. Substituierte Cyclohexen-derivate des Piperazins und Homopiperazins der allgemeinen Formel III

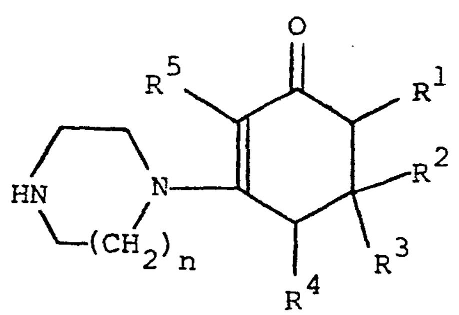

(III)

in der

$R^1$, $R^2$, $R^3$, $R^4$ und $R^5$, die gleich oder verschieden sein können, Wasserstoff, geradkettige oder verzweigte Alkylgruppen mit jeweils 1 bis 6 Kohlenstoffatomen, cyclische aliphatische Reste mit jeweils 5 bis 7 Kohlenstoffatomen, aromatische Reste oder substituierte aromatische Reste, die gleich oder verschieden mit Halogen, niederen Alkyl- oder Alkoxygruppen mit jeweils 1 bis 4 Kohlenstoffatomen im Alkylteil, einfach oder mehrfach substituiert sein können, Arylalkylreste, Furanylreste, Thienylreste oder

$R^2$ und $R^3$ zusammen die Gruppe $-(CH_2)_a-$, worin a die Zahl 4 oder 5 ist oder

$R^3$ und $R^4$ zusammen die Gruppe $-(CH_2)_b-$, worin b die Zahl 3, 4 oder 5 ist, bedeuten und

n die Zahl 1 oder 2 bedeutet,

und deren tautomere Formen

sowie deren Säureadditionssalze und Hydrate.

6. Verfahren zur Herstellung der Verbindungen der allgemeinen Formel I gemäß Anspruch 1, dadurch gekennzeichnet, daß man Chinazolin-Derivate der allgemeinen Formel II

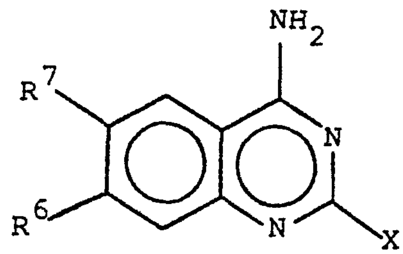

(II)

in der X Halogen, vorzugsweise Chlor oder Brom oder Alkylmercapto, vorzugsweise Methylmercapto ist und $R^6$ und $R^7$ die oben angegebene Bedeutung haben, mit Cyclohexenonderivaten des Piperazins oder Homopiperazins der allgemeinen Formel III

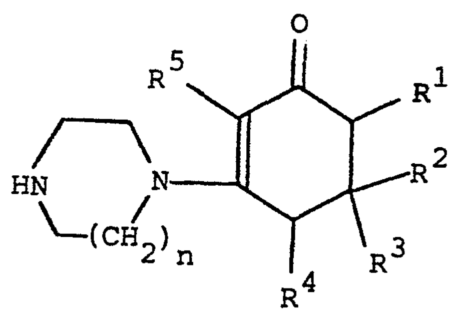

(III)

in der $R^1$ bis $R^5$ und n die obengenannte Bedeutung haben, umsetzt.

7. Verfahren zur Herstellung der Verbindungen der allgemeinen Formel I gemäß Anspruch 1, dadurch gekennzeichnet, daß man Verbindungen der allgemeinen Formel VI

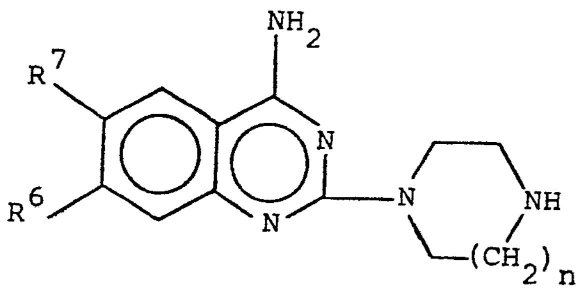

(VI)

in der $R^6$, $R^7$ und n die obengenannte Bedeutung haben, mit 1,3-Dioxocyclohexanen der allgemeinen Formel VII

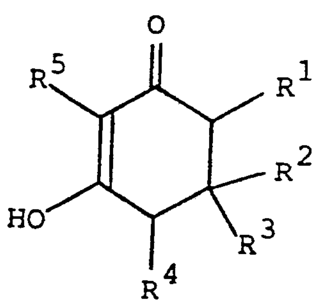

(VII)

in der $R^1$ bis $R^5$ die oben angegebene Bedeutung haben, umsetzt.

8. Verfahren zur Herstellung der Verbindungen der allgemeinen Formel III gemäß Anspruch 4, dadurch gekennzeichnet, daß man Piperazin oder Homopiperazin mit Verbindungen der allgemeinen Formel VII kondensiert.

9. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

10. Verbindungen der allgemeinen Formel I oder deren physiologisch verträgliche Säureadditionssalze zur Anwendung als Antihypertonica.

11. Verbindungen der allgemeinen Formel I oder deren physiologisch verträgliche Salze zur Behandlung von Herzinsuffizienz.

12. Verbindungen der allgemeinen Formel I oder deren physiologisch verträgliche Salze zur Glaukombehandlung.